Europäisches Patentamt

European Patent Office

Office européen des brevets

(19)

(11) Publication number : **0 611 575 A1**

## (12) EUROPEAN PATENT APPLICATION

(21) Application number : 94301050.4

(51) Int. Cl.⁵ : **A61L 15/58**

(22) Date of filing : 14.02.94

(30) Priority : **15.02.93 JP 47133/93**

(43) Date of publication of application :
**24.08.94 Bulletin 94/34**

(84) Designated Contracting States :
**AT BE DE DK ES FR IE IT LU MC NL SE**

(71) Applicant : **JOHNSON & JOHNSON
CONSUMER PRODUCTS, INC.
Grandview Road
Skillman, New Jersey 08558 (US)**

(72) Inventor : **Kumakura, Masahiro
1, Aza Meotozaka,
Oh-Aza Ohkanbara
Sukagawa-shi, Fukushima-ken (JP)**

(74) Representative : **Fisher, Adrian John et al
CARPMAELS & RANSFORD
43 Bloomsbury Square
London WC1A 2RA (GB)**

(54) **A pressure-sensitive adhesive composition for skin.**

(57)   A pressure-sensitive adhesive composition for skin comprising : 22-40 % by weight of a base composed substantially of an A-B-A type block copolymer wherein an A-block thereof is composed of styrene and/or styrene derivatives and a B-block thereof is composed of a conjugated diene and/or lower alkene, 40-60 % by weight of a tackifier, and 5-23 % by weight of a plasticizer.

EP 0 611 575 A1

EP 0 611 575 A1

The present invention relates to a pressure-sensitive adhesive composition for skin, and more specifically to a pressure-sensitive adhesive composition for skin containing as a base an A-B-A type block copolymer wherein an A-block thereof if composed of styrene and/or styrene derivatives and a B-block thereof is composed of a conjugated diene and/or lower alkene.

Pressure sensitive adhesives comprising a block copolymer of styrene, isoprene, etc. have been known. For example, U.S. Patent Nos. 3519585, 3676202, and 3787531 teaches such adhesives. Further, Japanese Patent Publication No. Sho 61-6872 discloses pressure-sensitive adhesives comprising a thermoplastic elastomer ingredient composed of an A-B type block copolymer and an A-B-A type block copolymer having excellent tackiness to the skin and to the oily surface.

The present inventors have further studied adhesives for skin, and have found that excellent tackiness to a wet surface is one of the most important factors for adhesives to the skin. Consequently they have found that adhesives comprising a specific A-B-A type block copolymer as a base, a tackifier and a plasticizer have excellent tackiness to a wet skin surface as well as a dry skin surface, and have attained the present invention.

The present invention, therefore, relates to a pressure-sensitive adhesive composition for skin comprising 22-40 % by weight of a base composed substantially of an A-B-A type block copolymer wherein an A-block thereof is composed of styrene and/or styrene derivatives and a B-block thereof is composed of a conjugated diene and/or lower alkene,

40-60 % by weight of a tackifier, and

5-23 % by weight of a plasticizer.

The base of the adhesive of the present invention comprises substantially an A-B-A type block copolymer wherein an A-block thereof is composed of styrene and/or styrene derivatives and a B-block thereof is composed of a conjugated diene and/or lower alkene.

The above styrene derivatives include compounds in which hydrogen(s) of a vinyl radical and/or benzene ring of styrene is(are) substituted with alkyl radical(s) or halogen atom(s), for example, alpha-methyl styrene and para-halogenated styrene.

The A-block of the A-B-A type block copolymer of the present invention is a homopolymer or copolymer of styrene and/or styrene derivatives, and the number average molecular weight of the A-block is preferably at least 7,000, more preferably in the range of 12,000-30,000.

The B-block of the A-B-A type block copolymer is a homopolymer or copolymer of a conjugated diene and/or lower alkene. The conjugated diene is a compound such as butadiene or isoprene. And the lower alkene includes an alkene whose carbon number is 6 or less, such as ethylene, propylene, butylene, pentene, hexene, etc. The amount of the A-block in the A-B-A type block copolymer is preferably 5-50 % by weight, and more preferably 10-30 % by weight. When said amount is less than 5 % by weight, it is not desirable because the adhesive may be too sticky to the skin. When it exceeds 50 % by weight, it is not desirable because the adhesion strength may be insufficient.

The number average molecular weight of the B-block is preferably 95,000-360,000. In this case, the number average molecular weight of the A-B-A type block copolymer is preferably 125,000-400,000.

The A-B-A type block copolymer includes so-called A-B type block copolymers, that is one of the A block of the A-B-A type block copolymer is hydrogen. Further, the A-B-A type block copolymer of the present invention includes so-called A-B-C type block copolymers, that is, two A blocks in the A-B-A type block copolymer are different with each other. But even in this case, both the A-block and the C-block must be composed of styrene and/or styrene derivatives. And the A-B-A type block copolymer of the present invention also includes block copolymers represented by the general formula: (A-B)n-X

wherein, an A-block is a homopolymer or copolymer of styrene and/or styrene derivatives, a B-block is a polymer of a conjugated diene such as butadiene or isoprene, and an X-block is an organic or inorganic substituent having two or more functional groups. Such block copolymers are described in US Patent No. 328183 and papers such as Chemical Week June 11, 1975 p.35 "New Rubber is backed by Stars.".

A total amount of styrene and/or styrene derivatives in the A-B-A type block copolymer of the present invention is preferably not more than 20 % by weight. When the total amount is more than 20 % by weight, the adhesive strength may be insufficient because the adhesive comes to be too hard.

The base of the adhesive of the present invention may include the other elastomer in the range of less than 25 % by weight. Such elastomer includes, for example, a natural rubber, styrene-butadiene rubber, butadiene rubber, isoprene rubber, acrylonitrile-butadiene rubber, and butyl rubber.

As the tackifier of the present invention, polymers which can give tackiness to the base or a mixture thereof may be used. Such tackifier includes polyterpene resins, aromatics-modified petroleum hydrocarbon resins, etc.

As the plasticizer of the present invention, any of the materials giving plasticity to the adhesive of the present invention may be used; such materials include a liquid rubber composed of isoprene, petroleum hydrocar-

2

bon resin, etc.

The present invention is based on the findings that the adhesive having excellent properties in adhesive strength, tackiness, cohesive force, and tackiness to the wet skin can be obtained, when three components, the base, tackifier and plasticizer are mixed in a specific ratio.

The tackiness of various compositions to wet and to dry skin is illustrated by the accompanying drawings, in which:

Figure 1 illustrates relations between compositions of three components and properties of the adhesive in dry condition.

Figure 2 illustrates the same in wet condition.

Figure 3 illustrates the area where the suitable compositions of the pressure-sensitive adhesive for skin can be obtained.

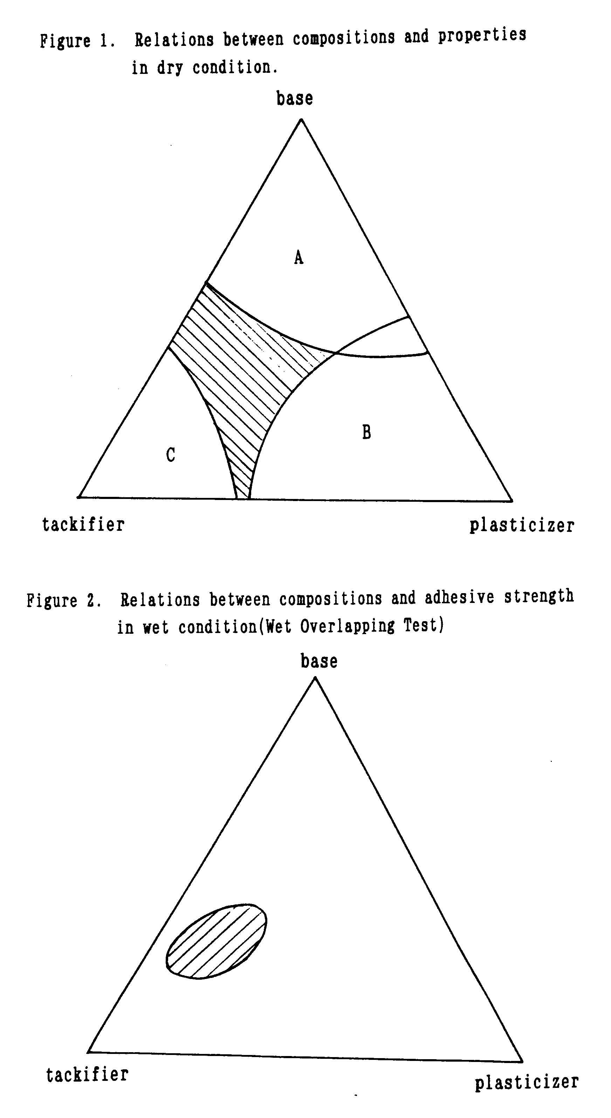

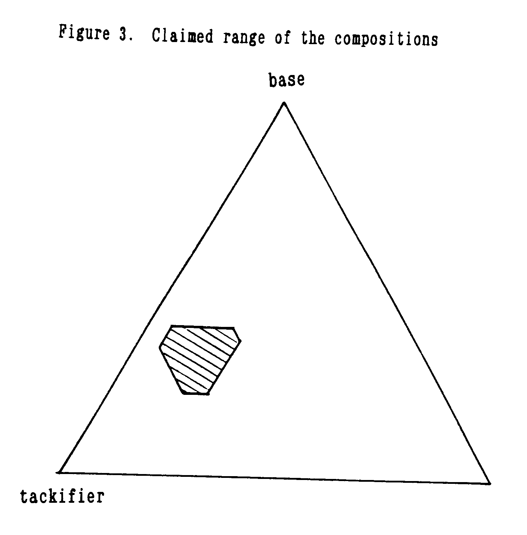

In the range A of Figure 1, the adhesion strength is too low, in the range B, the cohesive force is insufficient, and in the range C, the tackiness is insufficient. Accordingly, the adhesive in the ranges A, B and C is not preferable as the pressure-sensitive adhesive for skin. Further, outside the hatched area in Figure 2, the tackiness to the wet skin is insufficient. To sum up, the composition preferable for the pressure-sensitive adhesive for skin is an overlapping area of the hatched areas of both Figures A and B as follows:

| base | 22-40% by weight |
|------|------------------|
| tackifier | 40-60% by weight |
| plasticizer | 5-23% by weight |

The adhesive of the present invention usually contains less than 2% by weight of a known antioxidant. Such antioxidant includes, for example, 2,5-di-tert-amyl hydroquinone, di-tert-butyl cresol and 1,3,5-trimethyl-2,4,6-tris(3,5-di-tert-butyl-4-hydroxybenzyl)benzene.

The adhesive of the present invention may further contain a heat stabilizer such as zinc alkyldithiocarbamate, additives such as zinc oxide, aluminum hydrate, talc, calcium carbonate, titanium dioxide, carbon black, pigments, etc.

The following Examples are included merely to aid in the understanding of the invention, and variations may be made by one skilled in the art without departing from the spirit and scope of the invention.

In the following Examples, the compositions is expressed in terms of percent by weight based on the total amount of the base, tackifier and plasticizer.

The testing methods of adhesion strength (to silicone surface), adhesion strength (to skin), tackiness, and adhesion strength in wet condition in accordance with the present invention are as follows:

1. Test of adhesion strength to silicone surface:

An adhesive tape is adhered on a paper coated with silicone (Silicone SRX-370, TOREY·DOW CORNING Co.), and the adhesive tape and the paper are pressed together by a roll under the pressure of 4,500g. And tearing strength is measured when the tape is torn away from the paper at an angle of 180° at a rate of 300mm/min.

2. Test of adhesion strength to skin:

A adhesive tape is adhered to the skin inside the under arm of human body. And tearing strength is measured when the tape is torn away from the paper at an angle of 90° at the rate of 300mm/min.

3. Tackiness in a dry state:

Steal balls having diameter of from 1/16 to 1 inch are prepared at intervals of 1/16 inch, and a slope of an adhesive tape is given at an angle of 30° with a 10cm of an approach. The largest diameter of the steal ball which stops within 10cm on the adhesive tape is measured when the steal ball is rolled down on the slope of the adhesive tape after the 10cm free approach run.

4. Adhesive strength to a wet surface:

Samples are prepared by adhering an adhering side of the adhesive tape to the back of the tape, and are immersed into an aqueous solution of 0.75 % by weight of a powder-detergent, and the time (in minutes) that lapses until the sample tapes are torn away after applying a load of 200g is measured.

In the Tables, the base is a mixture of a styrene-isoprene block copolymer and a styrene-isoprene-styrene block copolymer, the tackifier is a polyterpene resin, and the plasticizer is a petroleum hydrocarbon resin.

In all the Examples listed above, 1.5 parts by weight of 1,3,5-trimethyl-2,4,6-tris(3,5-di-tert-butyl-4-hydroxybenzyl)b enzene per 100 parts by weight in total of the base, tackifier and plasticizer was added.

Examples 1-8

Pressure-sensitive adhesives having a composition shown in Table 1 were prepared, and the tests of adhesive strength to silicone, adhesive strength to skin and tackiness were performed. The results were listed in Table 1 and illustrated in Figure 1.

Table 1

| Experimental No. | 1 | 2 | 3 | 4 | 5 | 6 | 7 | 8 |
|---|---|---|---|---|---|---|---|---|
| base resin | 45.4 | 45.4 | 29.4 | 29.4 | 40.0 | 40.0 | 30.0 | 35.7 |
| tackifier | 27.3 | 50.0 | 35.3 | 64.7 | 60.0 | 50.0 | 60.0 | 46.4 |
| plasticizer | 27.3 | 4.6 | 35.3 | 5.9 | 0.0 | 10.0 | 10.0 | 17.9 |
| adhesive strength to silicone | 300 | 458 | 450 | >500 | >500 | 425 | >500 | 425 |
| adhesive strength to skin | 100 | 170 | 160 | 170 | 130 | 210 | 150 | -- |
| tackiness | 9/16 | 6/16 | 10/16 | 3/16 | 3/16 | 5/16 | 3/16 | 5/16 |

As shown in Table 1, Experiment Nos. 2, 5, 6, 7, and 8 have enough properties as an adhesive for skin.
The relations between the composition and properties are shown in Figure 1, wherein the hatched area is composition in which good properties as adhesives for skin can be obtained.

Example 2

To test adhesive strength in wet state, pressure-sensitive adhesives having a composition shown in Table 2 were prepared, and the test was performed. The results were listed in Table 2. Figure 1 illustrates the results of the Table 2.

Table 2

| Experimental No. | 9 | 10 | 11 | 12 | 13 | 14 | 15 |
|---|---|---|---|---|---|---|---|
| base resin | 45.4 | 45.4 | 29.4 | 29.4 | 40.0 | 40.0 | 30.0 |
| tackifier | 27.3 | 50.0 | 35.3 | 64.7 | 60.0 | 50.0 | 60.0 |
| plasticizer | 27.3 | 4.6 | 35.3 | 5.9 | 0.0 | 10.0 | 10.0 |
| adhesive strength in wet state | 3 | 13 | 13 | 65 | 17 | 10 | 36 |

Table 2 (continued)

| Experimental No. | 16 | 17 | 18 | 19 | 20 | 21 | 22 |
|---|---|---|---|---|---|---|---|
| base resin | 35.7 | 33.4 | 30.0 | 26.7 | 23.4 | 35.0 | 25.0 |
| tackifier | 46.4 | 50.0 | 55.0 | 60.0 | 65.0 | 55.0 | 55.0 |
| plasticizer | 17.9 | 16.6 | 15.0 | 13.3 | 11.6 | 10.0 | 20.0 |
| adhesive strength in wet state | 27 | 52 | 53 | 79 | 54 | 34 | 35 |

The adhesives having the adhesion strength of more than 20 are acceptable as adhesives for skin. Therefore as shown in Table 2, Experimental Nos. 12, 15, 16, 17, 18, 19, 20, 21 22 have enough properties as adhesives for skin.

The relations between the composition and properties are shown in Figure 2, wherein the hatched area indicates the composition having good properties as adhesives for skin can be obtained.

Specific ratios of the three components in the case of dried skin are illustrated in Figure 1 by the hatched area. The ratios outside this area are not preferable as pressure-sensitive adhesives for skin, because in the area shown by A, tackiness is too low, in the area shown by B, cohesive force and tackiness are not enough, and in the range shown by C, tackiness is not enough. And the specific ratio in the case of wet skin is illustrated by the hatched area in Figure 2, wherein sufficient adhesion strength can be obtained.

Consequently the suitable composition of the pressure-sensitive adhesive for skin is shown as the overlapping area of the hatched areas of Figure 1 and Figure 2. And this overlapping area is summarized as follows:

| the base: | 22-40 % by weight |
|---|---|
| the tackifier: | 44-60 % by weight |
| the plasticizer: | 5-23 % by weight |

This area is shown in Figure 3 as the hatched area.

Claims

1. A pressure-sensitive adhesive composition for skin comprising
    22-40% by weight of a base composed substantially of an A-B-A type block polymer wherein an A-block thereof is composed of styrene and/or styrene derivatives and a B-block thereof is composed of a conjugated diene and/or lower alkene,
    40-60% by weight of a tackifier, and
    5-23% by weight of a plasticizer.

2. A composition according to claim 1 wherein the number average molecular weight of the A-block is at least 7000.

3. A composition according to claim 1 or claim 2 wherein the amount of the A-block in the A-B-A type block copolymer is from 5 to 50% by weight, preferably 10 to 30% by weight.

4. A composition according to any preceding claim wherein the number average molecular weight of the B-block is from 95000 to 360000.

5

5. A composition according to any preceding claim wherein the number average molecular weight of the A-B-A type block copolymer is from 125000 to 400000.

6. A composition according to any preceding claim wherein the A-B-A type block copolymer is an A-B type block copolymer.

7. A composition according to any of claims 1 to 5 wherein the A-B-A type block copolymer is of the formula $(A-B)_n$-X, wherein an A-block is a homopolymer or copolymer of styrene and/or styrene derivatives, a B-block is a polymer of a conjugated diene and an X-block is an organic or inorganic substituent having two or more functional groups.

8. A composition according to any preceding claim wherein the total amount of styrene and/or styrene derivatives in the A-B-A type block copolymer is not more than 20% by weight.

9. A composition according to any preceding claim comprising no more than 25% by weight of an additional elastomer selected from a natural rubber, styrene-butadiene rubber, butadiene rubber, isoprene rubber, acrylonitrilebutadiene rubber and butyl rubber.

10. A composition according to any preceding claim further comprising a heat stabilizer and/or a pigment.

Figure 1. Relations between compositions and properties in dry condition.

Figure 2. Relations between compositions and adhesive strength in wet condition(Wet Overlapping Test)

Figure 3. Claimed range of the compositions

base

tackifier

| European Patent Office | **EUROPEAN SEARCH REPORT** | Application Number EP 94 30 1050 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X | EP-A-0 285 181 (TEIKOKU SEIYAKU KK)<br>* claims; examples * | 1-10 | A61L15/58 |
| X | EP-A-0 309 060 (AVERY INTERNATIONAL CORP.)<br>* page 3, line 18 - line 51; examples I-II * | 1-10 | |
| X | WO-A-91 02039 (THE DOW CHEMICAL COMPANY)<br>* claims * | 1-10 | |
| X | GB-A-2 045 618 (HISAMITSU PHARMACEUTICAL CO LTD.)<br>* page 1, line 37 - line 65; claims; examples * | 1-10 | |
| X | US-A-4 704 110 (GARY F. RAYKOVITZ ET AL.)<br>* claims * | 1-10 | |
| X | EP-A-0 428 017 (INDOPCO,INC.)<br>* the whole document * | 1-5,10 | |
| X | GB-A-2 089 351 (COLOPLAST A/S)<br>* claims * | 1 | TECHNICAL FIELDS SEARCHED (Int.Cl.5)<br><br>A61L |
| A | US-A-4 024 312 (RALF KORPMAN)<br>* column 2, line 17 - line 68 *<br>* column 3, line 1 - line 9 * | 1-10 | |

The present search report has been drawn up for all claims

| Place of search | Date of completion of the search | Examiner |
|---|---|---|
| THE HAGUE | 15 March 1994 | ESPINOSA, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document